# EUROPEAN PATENT SPECIFICATION

(11) **EP 3 145 559 B1**
(45) Date of publication and mention of the grant of the patent: **05.08.2020**
(21) Application number: 15753493.4
(22) Date of filing: 19.05.2015
(51) Int. Cl.: A61M 1/10, A61M 1/12

(54) **BLOOD PUMP**
BLUTPUMPE
POMPE À SANG

(30) Priority: 19.05.2014 US 201462000192 P
(43) Date of publication of application: 29.03.2017
(60) Divisional application: 20179137.3
(73) Proprietor: Magenta Medical Ltd., 60920 Kadima (IL)
(72) Inventor: SCHWAMMENTHAL, Ehud, 43411 Raanana (IL); TUVAL, Yosi, 40500 Even Yehuda (IL); GLOZMAN, Daniel, 90618 Kfar Adumim (IL)
(74) Representative: White, Duncan Rohan
(86) International application number: PCT/IL2015/050532
(87) International publication number: WO 2015/177793

(56) References cited:
- WO-A2-01/83016
- WO-A2-01/83016
- WO-A2-2005/020848
- WO-A2-2008/005747
- WO-A2-2008/005747
- AU-A1- 2013 205 145
- US-A1- 2007 208 291
- US-A1- 2007 208 291
- US-A1- 2014 128 659
- US-B1- 7 780 628

## Description

### FIELD OF EMBODIMENTS OF THE INVENTION

Some applications of the present invention generally relate to medical apparatus. Specifically, some applications of the present invention relate to apparatus and methods associated with placing a pump in one or more of a subject's renal veins, and/or in the subject's vena cava.

### BACKGROUND

It is common for cardiac dysfunction or congestive heart failure to develop into kidney dysfunction, which in turn, causes congestive heart failure symptoms to develop or worsen. Typically, systolic and/or diastolic cardiac dysfunction causes systemic venous congestion, which gives rise to an increase in renal venous and interstitial pressure. The increase in the pressure causes fluid retention by the body to increase due both to kidney dysfunction and renal neurohormonal activation, both of which typically develop as a result of the increase in renal venous and interstitial pressure. The resulting fluid retention causes congestive heart failure to develop or worsen, by causing a blood volume overload at the heart and/or by increasing systemic resistance. Similarly, it is common for kidney dysfunction and/or renal neurohormonal activation to develop into cardiac dysfunction and/or congestive heart failure. This pathophysiological cycle, in which cardiac dysfunction and/or congestive heart failure leads to kidney dysfunction and/or renal neurohormonal activation, or in which kidney dysfunction and/or renal neurohormonal activation leads to cardiac dysfunction and/or congestive heart failure, each dysfunction leading to deterioration in the other dysfunction, is called the cardio-renal syndrome.

Increased renal venous pressure has been experimentally shown to cause azotemia, and a reduction in glomerular filtration rate, renal blood flow, urine output, and sodium excretion. It has also been shown to increase plasma renin and aldosterone, and protein excretion. Venous congestion may also contribute to anemia via three different pathways: A reduction in the kidney's erythropoietin production, hemodilution by fluid retention, and an inflammatory response leading to a reduced gastro-intestinal iron uptake. Mechanistically, increased renal venous pressure may cause intracapsular pressure and, subsequently interstitial peritubular pressure, to rise. A rise in peritubular pressure may impact tubular function (reduce sodium excretion), as well as diminish glomerular filtration by raising the pressure in the Bowman capsule.

In heart failure patients, increased renal venous pressure may not only result from increased central venous (right atrial) pressure, but also from intraperitoneal fluid accumulations (ascites) exerting direct pressure on the renal veins. Reduction of intraabdominal pressure in heart failure patients by removal of fluid (e.g., via paracentesis, and/or ultrafiltration) has been shown to reduce plasma creatinine levels.

Increased venous return resulting from activation of the "leg muscle pump" during physical activity such as walking may raise systemic venous pressure, particularly in heart failure patients, and may result in reflux into the renal veins.

WO-A-0183016 discloses apparatus for treating congestive heart by actively of passively enhancing perfusion to the renal arteries. A specifically configured balloon catheter and extracorporeal pump, wherein the pump operates in a "once-through" fashion or alternating volume displacement mode.

US-A-2007/208291 discloses a catheter inserted into the aorta of the patient has an outlet port that supplies the patient's blood obtained from a patient's aorta into an adjoining renal artery of the patient to perfuse the kidney. The catheter comprises a rotor housed inside a portion of the catheter to supply the blood to the kidney at a desired pressure.

### SUMMARY OF EMBODIMENTS

In accordance with some applications of the present invention, a subject is identified as suffering from cardiac dysfunction, congestive heart failure, reduced renal blood flow, increased renal vascular resistance, arterial hypertension, diabetes, and/or kidney dysfunction. In response thereto, blood pressure within the subject's renal veins is reduced by placing at least one pump in the subject's vena cava, and generating a low- pressure region within the subject's vena cava adjacent to junctions of the vena cava with the subject's renal veins, by activating the pump to pump blood away from the region. The pump is activated such that blood pressure within the low-pressure region is lower than central venous pressure of the subject. Typically, a downstream pump is placed within the vena cava downstream of the junctions of the vena cava with the subject's renal veins, and the pump pumps blood through the vena cava in the downstream direction, away from the junctions. For some applications, an upstream pump is placed within the vena cava upstream of the junctions of the vena cava with the subject's renal veins, and the pump pumps blood through the vena cava in the upstream direction, away from the junctions. Alternatively or additionally, an occlusion element, such as a balloon or a covered stent is placed in the vena cava upstream of the junctions, and is configured to partially occlude the vena cava upstream of the junctions.

For some applications, the upstream and downstream pumps are disposed on a single catheter. Typically, the catheter is inserted into the vena cava via a venous pathway, e.g., via the femoral vein, via the subclavian vein, or via the jugular vein. For some applications, the upstream pump, or the occlusion element is disposed on a first catheter, which is inserted via a vein that is below the subject's inferior vena cava (e.g., the femoral vein), and the downstream pump is disposed on a second catheter, which is inserted via a vein that is above the subject's inferior vena cava (e.g., the subclavian vein, or the jugular vein).

For some applications, the downstream pump and/or the upstream pump includes an impeller and a cage. For some applications, impellers of the downstream and the upstream pumps rotate in the same direction, but the downstream pump is configured to pump blood in the downstream direction and the upstream pump is configured to pump blood in the upstream direction. For some such applications, a single motor is used to impart rotational motion to both of the impellers, and there is a shaft disposed between the impellers that imparts rotational motion from a first one of the impellers to a second one of the impellers. Typically, for such applications, the impellers of the upstream and the downstream pumps are (a) of opposing handedness with respect to one another (i.e., one of the impellers is a left-handed impeller, and the other impeller is a right-handed impeller), and (b) are disposed upon the aforementioned shaft, such that the impellers are facing opposite directions to one another.

In general, in the specification and in the claims of the present application, the term "proximal" and related terms, when used with reference to a device or a portion thereof, should be interpreted to mean an end of the device or the portion thereof that, when inserted into a subject's body, is typically closer to a location through which the device is inserted into the subject's body. The term "distal" and related terms, when used with reference to a device or a portion thereof, should be interpreted to mean an end of the device or the portion thereof that, when inserted into a subject's body, is typically further from the location through which the device is inserted into the subject's body. In general, in the specification and in the claims of the present application, the term
"downstream" and related terms, when used with reference to a blood vessel, or with reference to a portion of a device that is configured to be placed inside a blood vessel, should be interpreted to mean a location within the blood vessel, or a portion of the device that is intended for placement at a location within the blood vessel, that is downstream, with respect to the direction of antegrade blood flow through the blood vessel, relative to a different location within the blood vessel. The term "upstream" and related terms, when used with reference to a blood vessel, or with reference to a portion of a device that is configured to be placed inside a blood vessel, should be interpreted to mean a location within the blood vessel, or a portion of the device that is intended for placement at a location within the blood vessel, that is upstream with respect to the direction of antegrade blood flow through the blood vessel, relative to a different location within the blood vessel.

The invention provides an apparatus as claimed in claim 1.

The present invention will be more fully understood from the following detailed description of embodiments thereof, taken together with the drawings, in which:

### BRIEF DESCRIPTION OF THE DRAWINGS

Figs. 1A-D are schematic illustrations of a blood-pump catheter placed within a subject's vena cava, an upstream pump being disposed upon the catheter, distally to a downstream pump, in accordance with some applications of the present invention;
Fig. 2 is a schematic illustration of the catheter of Figs. 1A-D inserted into the subject's vena cava via the subject's right jugular vein, in accordance with some applications of the present invention;
Fig. 3 is a schematic illustration of a blood-pump catheter inserted into a subject's vena cava via the subject's femoral vein, a downstream pump being disposed upon the catheter distally to an upstream pump, in accordance with some applications of the present invention;
Fig. 4 is a schematic illustration of upstream and downstream pumps disposed on respective blood-pump catheters, in accordance with some applications of the present invention;
Figs. 5A-B are schematic illustrations of a catheter that includes a downstream pump and an occlusion element, such as a balloon (Fig. 5A), or a covered frame (Fig. 5B), in accordance with some applications of the present invention; and
Fig. 6 is a schematic illustration of a blood-impermeable sleeve configured to occlude blood flow from a subject's vena cava to the subject's renal veins, as described in WO14/141284, and in accordance with some applications of the present invention.

### DETAILED DESCRIPTION OF EMBODIMENTS

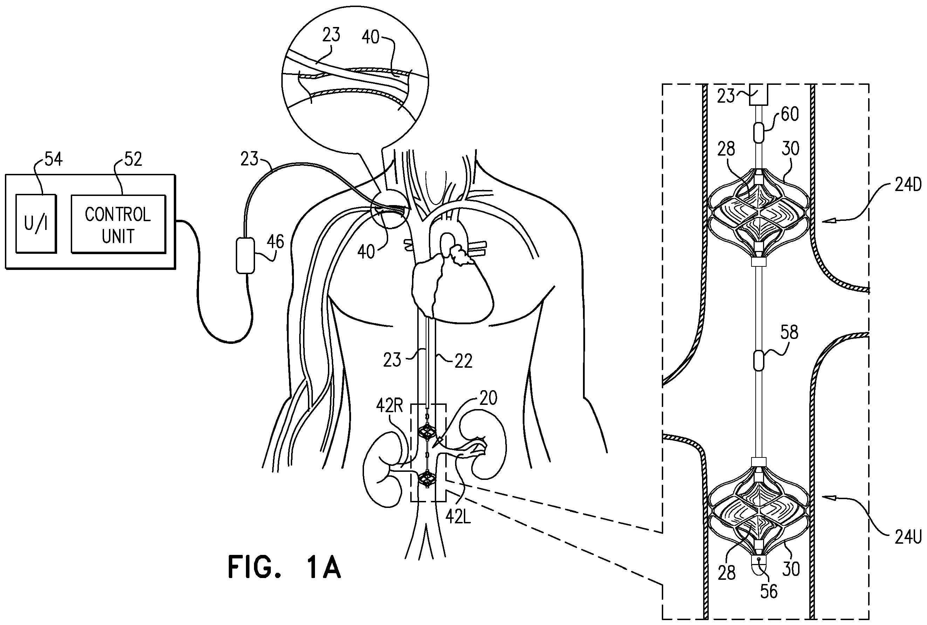

Reference is made to Figs. 1A-D, which are schematic illustrations of a blood-pump catheter 20 placed within a subject's vena cava 22, via a guide catheter 23, an upstream pump 24U being disposed upon the catheter, distally to a downstream pump 24D, in accordance with some applications of the present invention. Typically, the distal portion of blood-pump catheter 20 is configured to be straight, when the catheter is in a non-constrained state, such that both the upstream and the downstream pumps are disposed along the axis of the catheter, within the vena cava.

Each of the upstream and downstream pumps 24U and 24D typically includes a radially-expandable impeller 28 disposed inside a radially-expandable impeller cage 30. Typically, impeller 28 and cage 30 are shape set such as to assume radially-expanded configurations thereof in the absence of any radially-constraining force acting upon the impeller and the cage. Further typically, an engagement mechanism engages the impeller and the cage with respect to one another, such that in response to the cage becoming radially constrained the impeller becomes radially constrained, e.g., in accordance with apparatus and methods described in described in WO 14/141284 to Schwammenthal.

It is noted that the term "impeller" is used herein to denote a bladed rotor, as shown in 1A-D, for example. When the bladed rotor is placed inside a blood vessel (such as vena cava 22) and rotated, the bladed rotor functions as an impeller, by modifying the flow of blood through the blood vessel, and/or by generating a pressure difference between the upstream end and the downstream end of the impeller.

It is noted that reference numeral 24 is generally used to denote a blood pump in the present application. When a pump that is placed upstream is being referred to, reference numeral 24U is used, and when a pump that is placed downstream is being referred to, reference numeral 24D is used. Similarly, reference numeral 28 is generally used to denote an impeller in the present application. When an impeller that is placed upstream is being referred to, reference numeral 28U is used, and when an impeller that is placed downstream is being referred to, reference numeral 28D is used.

Blood-pump catheter 20 is typically placed inside the subject's vena cava 22, and operated therein, in order to provide acute treatment of a subject suffering from cardiac dysfunction, congestive heart failure, low renal blood flow, high renal vascular resistance, arterial hypertension, diabetes, and/or kidney dysfunction. For example, the blood-pump catheter may be placed inside the subject's vena cava, and operated therein, for a period of more than one hour (e.g., more than one day), less than one week (e.g., less than four days), and/or between one hour and one week (e.g., between one day and four days). For some applications, the blood-pump catheter is chronically placed inside the subject's vena cava in order to provide chronic treatment of a subject suffering from cardiac dysfunction, congestive heart failure, low renal blood flow, high renal vascular resistance, arterial hypertension, diabetes, and/or kidney dysfunction. For some applications, a course of treatment is applied to a subject over several weeks, several months, or several years, during which the blood-pump catheter is intermittently placed inside the subject's vena cava, and the subject is intermittently treated in accordance with the techniques described herein. For example, the subject may be intermittently treated at intervals of several days, several weeks, or several months.

For some applications, blood-pump catheter 20 is inserted into vena cava 22, via the subject's subclavian vein 40, as shown in Fig. 1A. Typically, the blood-pump catheter is inserted under fluoroscopic imaging. Alternatively, the blood-pump catheter is inserted under ultrasound imaging, such as to reduce exposure of the subject to radiation and/or contrast agent. The catheter is placed into the vena cava such that upstream pump 24U is disposed upstream of the junctions of the vena cava and all of the subject's renal veins 42, and such that downstream pump 24D is disposed downstream of the junctions of the vena cava and all of the subject's renal veins. Typically, the upstream pump is configured to pump blood through the vena cava in the upstream direction, away from the renal veins, and the downstream pump is configured to pump blood through the vena cava in the downstream direction, away from the renal veins.

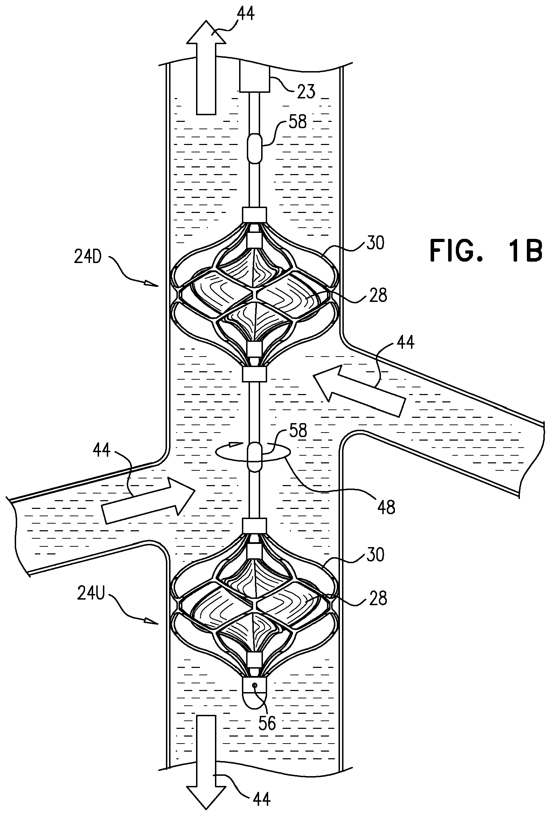

The effect of both of pumps 24U and 24D pumping blood in the above-described manner is that, between the pumps, and adjacent to the junctions of the vena cava with the renal veins, there is a low-pressure region of the vena cava, within which blood pressure is lower than the subject's central venous pressure. Functionally, this region may be viewed as a compartment within the vena cava within which blood pressure is controlled (by controlling pumps 24U and 24D), regardless of the blood pressure elsewhere within the vena cava. This typically increases blood flow from the renal veins into the vena cava, lowers pressure within the subject's renal veins, and causes renal perfusion to increase. The effect of pumps 24U and 24D on blood flow through the renal veins and the vena cava is indicated by arrows 44 in Fig. 1B.

As described hereinabove, the effect of operating blood pumps 24U and 24D is that between the pumps there is a low-pressure region of the vena cava. However, typically, the pumps are operated simultaneously such that the pressure within other portions of the vena cava is substantially unchanged relative to when blood-pump catheter 20 is not in operation. For example, the pumps are typically operated simultaneously such that the pressure within the vena cava downstream of downstream pump 24D is not substantially increased relative to when blood-pump catheter 20 is not in operation. Similarly, the pumps are typically operated simultaneously such that the pressure within the vena cava upstream of upstream pump 24U is not substantially increased relative to when blood-pump catheter 20 is not in operation. This is because the pumps are typically operated simultaneously such that outside of the region between the two pumps, the effects of the pumping by the upstream and downstream pumps cancel each other with respect to pressure. It is noted that there is likely to be some increase in the pressure within the vena cava downstream of downstream pump and upstream of upstream pump due to the increased blood flow from the renal veins into the vena cava.

Similarly, the pumps are typically operated simultaneously such that venous return to the vena cava from regions upstream of the upstream pump and downstream from the downstream pump is substantially unchanged relative to when blood-pump catheter 20 is not in operation. In this manner, the pumps the pumps are typically operated simultaneously such as to have a generally synergistic effect on pressure and flow in the region between the pumps, but to have an antagonistic effect on pressure and flow outside of the region, such that, outside of the region, the effects of the two pumps typically substantially cancel each other.

Typically, blood-pump catheter 20 pumps blood in a manner that enhances the rate of flow of blood flow through the renal veins and into the vena cava, but does not cause a substantial change in the direction of the blood flow relative to the natural direction of flow through the renal veins, or from the renal veins to the vena cava (i.e., relative to blood flow in the absence of pumping by the blood-pump catheter). That is to say that the blood-pump catheter pumps blood in the downstream direction through the renal veins and then directly into the portion of the vena cava that is adjacent to the renal veins, rather than, for example, pumping the blood from the renal veins into a different portion of the subject's veins (such as, an upstream location within the vena cava). It is noted that, due to the pumping of the downstream pump in the downstream direction, there is likely to be some blood flow from the renal veins to the portion of the vena cava that is below the renal veins. Further typically, blood-pump catheter 20 enhances blood flow through the renal veins without removing blood from the subject's venous system into a non-venous receptacle, such as an artificial lumen of a blood pump.

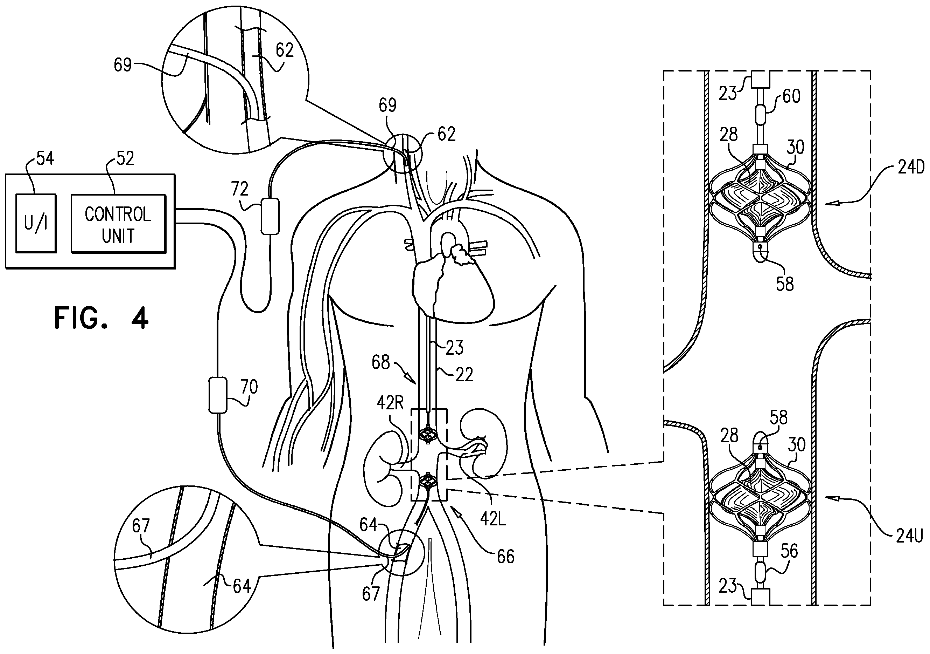

As described hereinabove, typically blood-pump catheter 20 is placed inside the vena cava of a subject suffering from cardiac dysfunction, congestive heart failure, low renal blood flow, high renal vascular resistance, arterial hypertension, diabetes, and/or kidney dysfunction. Typically, operating the blood-pump catheter in the vena cava of such a subject causes a lowering and flattening of the subject's renal vein pressure profile, even though the subject's central venous pressure is elevated, e.g., as described with reference to Fig. 4B of WO 14/141284 to Schwammenthal.

Typically, due to the reduction in pressure in the renal vein that is caused by the pumping of blood by blood-pump catheter 20, perfusion of the kidney increases. In turn, this may cause pressure in the renal veins to rise relative to the pressure in the renal veins immediately subsequent to initiation of the pumping, due to increased blood flow into the renal vein. Typically, even after perfusion of the kidney increases, the pump is configured to maintain the pressure in the renal vein at a lower value than the pressure in the renal vein before the initiation of the pumping. For some applications, in addition to lowering the subject's renal vein pressure, and/or increasing perfusion of the subject's kidney, blood-pump catheter 20 performs ultrafiltration on the subject's blood.

It is noted that, for some applications, due to the reduction in pressure in the renal vein that is caused by the pumping of blood by blood-pump catheter 20, the subject's renal vascular resistance decreases, in accordance with physiological mechanisms that are described, for example, in an article by Haddy et al., entitled "Effect of elevation of intraluminal pressure on renal vascular resistance" (Circulation Research, 1956). It is further noted that a treatment of the subject that increases renal perfusion by increasing blood pressure in the subject's renal arteries would typically not effect the aforementioned physiological mechanisms.

Typically, when blood-pump catheter 20 is used to reduce pressure in the subject's renal veins, it is expected that there will be an improved responsiveness by the subject to administration of diuretics to the subject, due to the reduction in renal venous pressure. Therefore, for some applications, a reduced dosage of diuretics may be administered to the subject relative to a dosage of diuretics that would be administered to the subject in the absence of performing the techniques described herein. Alternatively, a regular dosage of diuretics may be administered to the subject, but the diuretics may have a greater effect on the subject, due to the reduction in renal venous pressure.

Typically, high central venous pressure leads to a high level of blood pressure within the heart, which in turn leads to the release of atrial natriuretic peptide (ANP) and B-type natriuretic peptide (BNP) by the subject, both of which act as natural diuretics. For some applications, when blood-pump catheter 20 is used to reduce pressure in the subject's renal veins, there is expected to be an improved responsiveness by the subject to the release of the natural diuretics by the subject, due to the reduction in renal venous pressure. For some applications, since the subject's central venous pressure is not lowered by using blood-pump catheter 20, it is expected that the subject will continue to release atrial natriuretic peptide (ANP) and B-type natriuretic peptide (BNP), even while the subject's renal venous pressure is reduced by the use of the blood pumps. Thus, for some applications, using blood-pump catheter 20 may result in the subject continuing to release atrial natriuretic peptide (ANP) and B-type natriuretic peptide (BNP), as well as resulting in the effectiveness of the aforementioned natural diuretics being greater than the effectiveness of the diuretics in the absence of the use of blood-pump catheter 20.

Typically, each of upstream and downstream pumps 24U and 24D includes an impeller 28, for example, any one of the impellers described in WO 14/141284 to Schwammenthal. In accordance with respective applications, impeller 28 may have a single blade, two blades (e.g., as described in WO 14/141284 to Schwammenthal), three blades (e.g., as described in WO 14/141284 to Schwammenthal), or more than three blades. For some applications, one or both of blood pumps 24U and 24D includes more than one impeller. Typically, *ceteris paribus,* by using more than one impeller in at least one of the pumps, in order to generate a given flow of blood with the pump, the force that impacts each of the impellers within the pump is smaller than if a single impeller were to be used in the pump.

For some applications, one or both of the pumps includes radially-expandable cage 30. Typically, cage 30 is configured to hold open the inner wall of the vena cava and to separate the inner wall of the vena cava from the impeller, such that the vena cava does not become injured by the impeller. As described hereinabove, typically, impeller 28 and 30 are shape set such as to assume radially-expanded configurations thereof in the absence of any radially-constraining force acting upon the impeller and/or the cage. Further typically, an engagement mechanism engages the impeller and the cage with respect to one another, such that in response to the cage becoming radially constrained the impeller becomes radially constrained, e.g., in accordance with apparatus and methods described in described in WO 14/141284 to Schwammenthal.

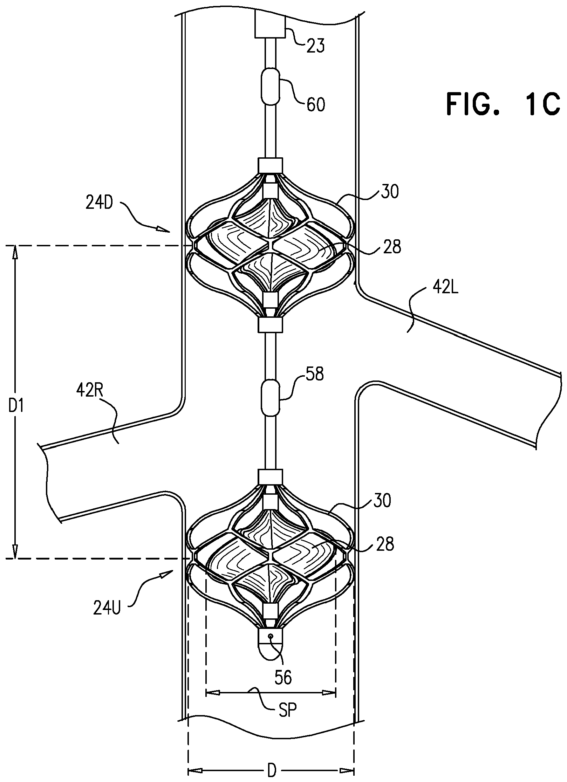

Referring now to Fig. 1C, typically, when blood-pump catheter 20 is placed inside vena cava 22, impeller 28 and cage 30 are substantially not radially constrained, due to the relatively low radial force exerted by the vena cava wall on the cage. Typically, a span SP of impeller 28, when the impeller is in a non-constrained configuration thereof inside the vena cava is more than 14 mm (e.g., more than 16 mm), and/or less than 28 mm (e.g., less than 22 mm), e.g., 14-28 mm, or 16-22 mm. Typically, a diameter D of cage 30, when the cage is in a non-constrained configuration thereof inside the vena cava is more than 14 mm (e.g., more than 16 mm), and/or less than 40 mm (e.g., less than 35 mm), e.g., 14-40 mm, or 16-35 mm. Further typically, when blood-pump catheter 20 is used to enhance blood flow from the renal veins into the subject's vena cava, as described herein, a longitudinal distance D1 between centers of the impellers of the upstream and downstream pumps, measured along the longitudinal axis of the catheter, is typically more than 3 cm (e.g., more than 6 cm), and/or less than 18 cm (e.g., less than 14 cm), e.g., 3-18 cm, or 6-14 cm.

Typically, impellers of pumps 24U and 24D are coupled to one or more motors 46 (Fig. 1A), which impart rotational motion to the impellers, via one or more shafts, the shaft(s) being housed inside blood-pump catheter 20. In accordance with respective applications, the motors are disposed outside of the subject's body (as shown), or are placed inside the subject's body (not shown).

For some applications, in order for the impellers to pump blood in opposite directions (i.e., in order for the upstream impeller to pump blood upstream, and the downstream pump to pump blood downstream), the impellers are rotated in opposite directions from one another, as viewed from an external reference point.

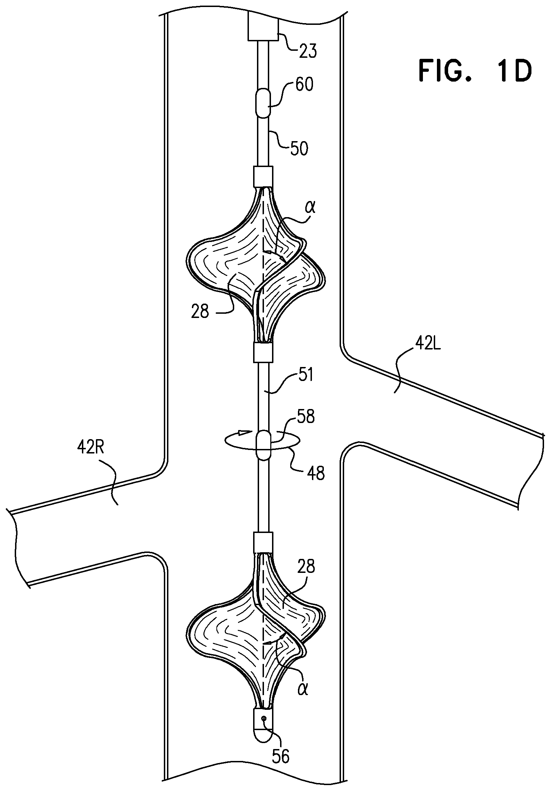

Referring now to Fig. 1D, typically, impellers 28 of upstream and downstream pumps 24U and 24D are rotated in the same rotational direction as one another, as viewed from an external reference point (e.g., in the direction of arrow 48 (i.e., clockwise), or counterclockwise), but the impellers are disposed on the catheter such that the rotation of the impellers in this direction of rotation causes the impellers to pump blood in respective, opposite directions. It is noted that the rotational direction of the impellers "as viewed from an external reference point" should be interpreted to mean the direction of rotational motion of the impellers as observed from any point that is not undergoing the same rotational motion as either of the impellers. (For illustrative purposes, Fig. 1D shows the impellers in the absence of the cages, although typically, the impellers are used together with cages, as described hereinabove.)

Typically, for such applications, a single motor is used to rotate both of the impellers. A shaft 50 is used to impart the rotational motion from the motor to the proximal impeller. An additional shaft 51, which is in series with shaft 50, couples the proximal impeller to the distal impeller and imparts the rotational motion from the proximal impeller to the distal impeller. For some applications, by using a single series of shafts to impart rotation to impellers 28 of both upstream and downstream pumps 24U and 24D, the diameter of blood-pump catheter 20 is reduced relative to if parallel shafts were used, in order to impart rotation to the upstream and downstream impellers.

For some applications, the angles and/or orientations of the impeller blades of impellers 28 of upstream and downstream pumps 24U and 24D may be such as to cause the impellers to pump blood in respective, opposite directions. For some applications, as shown in Fig. 1D, each propeller is shaped and/or oriented in the mirror image of the other, the axis of reflection being orthogonal to the longitudinal axes of the impellers. Typically, the upstream and downstream impellers are of opposing-handedness to one another, a first one of the impellers being a left-handed impeller, and the other one of the impellers being a right-handed impeller. It is generally the case that impellers of opposing handedness that are positioned parallel to one another, facing the same direction as one another, and rotating in opposite rotational directions from one another, generate flow in the same direction as one another. In accordance with the present invention, the upstream and downstream impellers are typically disposed upon shaft 51 such that the impellers are facing in opposite directions to one another. As described hereinabove, the impellers are typically rotated in the same rotational direction as one another, as viewed from an external reference point. The result of the impellers (a) being of opposing handedness to one another, and (b) facing in opposite directions, is that, when the impellers are rotated in the same direction as one another about an axis defined by shaft 51, the impellers pump blood in opposite directions from one another.

Typically, the blades of the downstream impeller are oriented such that, as the downstream impeller rotates in the direction of arrow 48, the downstream impeller pumps in the downstream direction. The blades of the upstream impeller are oriented such that, as the upstream impeller rotates in the direction of arrow 48, the upstream impeller pumps in the upstream direction.

As described in further detail hereinbelow, for some applications, upstream and downstream pumps 24U and 24D and blood-pump catheter 20 are placed within a main artery upstream and downstream of bifurcations of the artery with one or more branching arterial systems that branch from the main artery and supply a given organ, *mutatis mutandis.* For such applications, the blades of the downstream impeller are oriented such that, as the downstream impeller is rotated, the downstream impeller pumps in the upstream direction (toward the bifurcations). The blades of the upstream impeller are oriented such that, as the upstream impeller rotates is rotated, the upstream impeller pumps in the downstream direction (toward the bifurcations), such that blood flow into the branching arterial system is increased, thereby increasing perfusion of the organ.

For some applications, the blades of the impellers of the upstream and downstream pumps are configured to pump blood in the same direction as one another (e.g., in the antegrade direction). For example, the impellers may be of the same handedness as one another, placed upon catheter 20 such that the impellers are facing in the same direction as one another, and rotated in the same direction as one another, as viewed from an external reference point. Alternatively, the two impellers may be of opposing handedness to one another, placed within the vena cava such that the two impellers are facing in the same direction as one another, and rotated in opposite directions to one another, as viewed from an external reference point.

For some applications, blades of the upstream and downstream impellers are disposed at an angle *alpha* with respect to the longitudinal axes of the impellers, the blades of the respective impellers being oriented in opposite directions. For some applications, angle *alpha* is greater than 15 degrees (e.g., greater than 25 degrees), and/or less than 45 degrees (e.g., less than 35 degrees), e.g. 15-45 degrees, or 25-35 degrees.

For some applications, impellers 28 of upstream and downstream pumps 24U and 24D are rotated at respective rotation rates, in order to cause the pumping of blood in the upstream and downstream directions to be performed at respective rates. Alternatively, the impellers are rotated at the same rotation rate (and, typically, in the same direction), but the impellers are sized, shaped, and/or oriented such that the rate at which blood is pumped, respectively, in the upstream and downstream directions, by the respective impellers, is not equal.

Typically, a control unit 52 and a user interface 54 are disposed outside the subject's body. Further typically, the control unit receives inputs from one or more pressure sensors 56, 58, and/or 60, e.g., as shown in Figs. 1A-D.

In accordance with some applications:
(a) a pressure sensor 56 is disposed on the upstream side of upstream blood pump 24U and is configured to measure pressure within the vena cava upstream of the low-pressure region of the vena cava, which is typically indicative of venous pressure within the subject's lower body;
(b) a pressure sensor 58 disposed between the two blood pumps, and is configured to measure pressure within the low-pressure region of the vena cava between the two blood pumps, which is typically indicative of blood pressure within the subject's renal veins; and/or
(c) a pressure sensor 60 is disposed on the downstream side of downstream blood pump 24D and is configured to measure pressure within the vena cava downstream of the low-pressure region of the vena cava, which is typically indicative of the subject's central venous pressure close the subject's right heart.

For some applications, blood-pump catheter 20 includes pressure sensor 58 disposed between the two blood pumps, and is configured to measure pressure within the low-pressure region of the vena cava between the two blood pumps, which is typically indicative of blood pressure within the subject's renal veins, and the blood-pump catheter does not include pressure sensor 56, or pressure sensor 60.

For some applications, control unit 52 controls pumps 24U and 24D, e.g., by controlling rotation of impellers 28, responsively to one or more of the above-described inputs. Typically, user interface 54 displays the subject's current lower-body venous pressure, renal venous pressure, and/or central venous pressure, based upon the signals generated by sensors 56, 58, and/or 60. Typically, based upon the current values of the subject's lower-body venous pressure, renal venous pressure, and/or central venous pressure, a user (such as a healthcare professional) inputs a target value for the subject renal venous pressure, via the user interface. In response thereto, control unit 52 controls the speed of the rotation of the impellers, such that the impellers pump blood away from the renal veins at a flow rate that is such as to reduce the renal venous pressure toward the target level, as indicated by the user. For some applications, in response a signal received from sensor 60 indicating that the central venous pressure is at the target renal venous pressure, the control unit stops the impellers rotating. For some applications, the control unit receives an input from an additional sensor (such as a flow sensor and/or an oxygen-saturation sensor, and/or a thermal flow sensor, e.g., as described with reference to Figs. 22Ai-22Cii of WO 14/141284 to Schwammenthal), and the control unit controls the speed of the rotation of the impellers responsively to an input from the additional sensor.

It is noted that control unit 52 typically includes a computer processor that comprises circuitry and that is configured to execute the actions described herein. Typically, the operations described herein that are performed by the computer processor transform the physical state of a memory, which is a real physical article that is in communication with the computer processor, to have a different magnetic polarity, electrical charge, or the like depending on the technology of the memory that is used. Control unit 52 is typically a hardware device programmed with computer program instructions to produce a special purpose computer. For example, when programmed to perform the techniques described herein, control unit 52 typically acts as a special purpose renal-venous-pressure-modulating computer processor.

It is further noted that user interface 54 typically includes any type of user interface configured to receive inputs from a user and/or to provide outputs to the user. For example, the user interface may include one or more input devices (such as a keyboard, a mouse, a trackball, a joystick, a touchscreen monitor, a touchpad, a voice-command interface, a smartphone, a tablet computer, and/or other types of input devices that are known in the art), and/or one or more output devices (such as a monitor, an audio output device, a smartphone, a tablet computer, and/or other types of output devices that are known in the art).

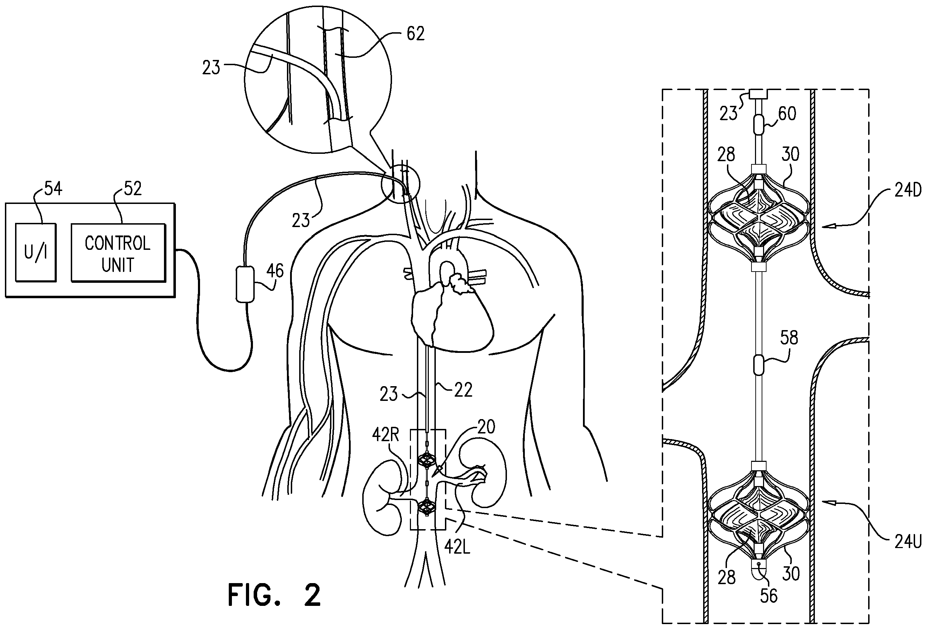

Reference is now made to Fig. 2, which is a schematic illustration of blood-pump catheter 20 being inserted into the subject's vena cava 22 via the subject's right jugular vein 62 (through guide catheter 23), in accordance with some applications of the present invention. For some applications, instead of being inserted via the subclavian vein (as shown in Fig. 1A, for example), blood-pump catheter 20 is inserted into the vena cava via the subject's right jugular vein, or via another vein that is above the subject's inferior vena cava. In all other aspects, blood-pump catheter 20 and the functioning thereof are generally as described with reference to Figs. 1A-D.

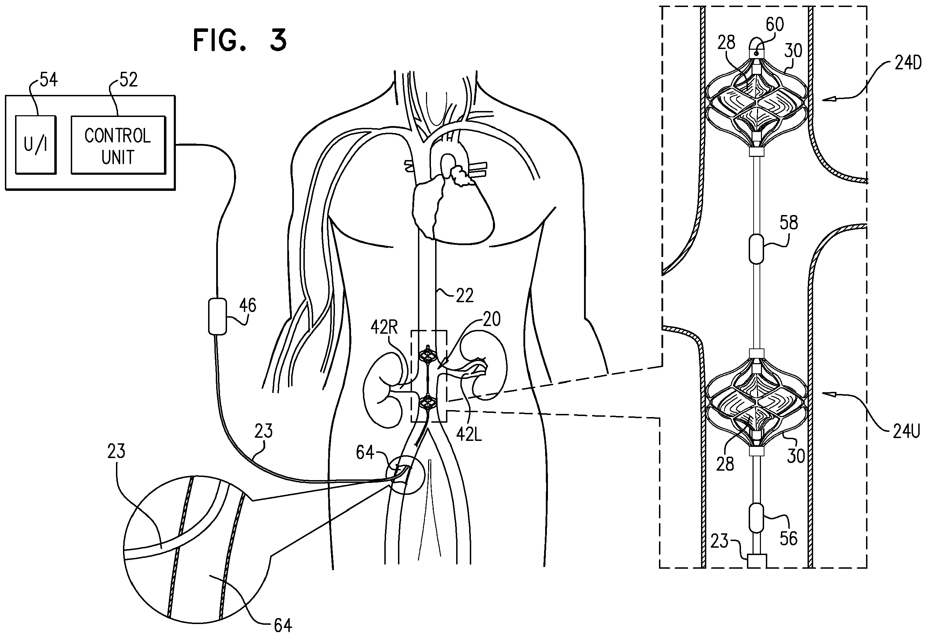

Reference is now made to Fig. 3, which is a schematic illustration of blood-pump catheter 20 being inserted into the subject's vena cava 22 via the subject's femoral vein 64 (through guide catheter 23), downstream pump 24D being disposed upon the catheter distally to upstream pump 24U, in accordance with some applications of the present invention. For some applications, instead of being inserted via the subclavian vein (as shown in Fig. 1A, for example), blood-pump catheter 20 is inserted into the vena cava, via the subject's femoral vein 64, or via another vein that is below the subject's inferior vena cava. Typically, downstream blood pump 24D is disposed on blood-pump catheter 20 distally to upstream blood pump 24U. Blood-pump catheter 20 is configured to be placed within the vena cava, such that the upstream pump is disposed upstream of the junctions of the vena cava with all of the subject's renal veins 42, and such that the downstream pump is disposed downstream of the junctions of the vena cava with all of the subject's renal veins. Other than the dispositions of the upstream and downstream blood pumps with respect to blood-pump catheter 20, blood-pump catheter 20, as shown in Fig. 3, and the functioning thereof, is generally similar to that described with reference to blood-pump catheter 20 as shown in Figs. 1A-D.

Reference is now made to Fig. 4, which is a schematic illustration of upstream and downstream pumps 24 U and 24D being disposed on respective catheters 66 and 68, in accordance with some applications of the present invention. For some applications, a first catheter 66 is inserted into vena cava 22 through a guide catheter 67 that is inserted via the subject's femoral vein 64, or via another vein that is below the subject's inferior vena cava. Upstream blood pump 24U is disposed on the first catheter, and is configured to be placed within the vena cava upstream of the junctions of the vena cava with all of the subject's renal veins, and to pump blood through the vena cava in the manner described hereinabove. A second catheter 68 is inserted into the vena cava through a guide catheter 69 that is inserted via the subject's jugular vein 62 (as shown), via the subclavian vein (not shown), or via a different vein that is above the subject's inferior vena cava. Downstream blood pump 24D is disposed on the second catheter, and is configured to be placed within the vena cava downstream of the junctions of the vena cava with all of the subject's renal veins, and to pump blood through the vena cava in the manner described hereinabove.

For applications in which the upstream and downstream blood pumps include impellers, typically, respective motors 70 and 72 are used to control rotation of the impellers. Further typically, control unit 52 controls both pumps (e.g., by controlling the rates of rotation of the impellers). For some applications, pressure sensors 56, 58 and 60 are disposed upon the first and/or second catheters, and are configured to detect indications of, respectively, lower body venous pressure, renal venous pressure, and central venous pressure. The control unit is configured to control the operation of the upstream and downstream pumps responsively to the detected indications, in accordance with the techniques described hereinabove.

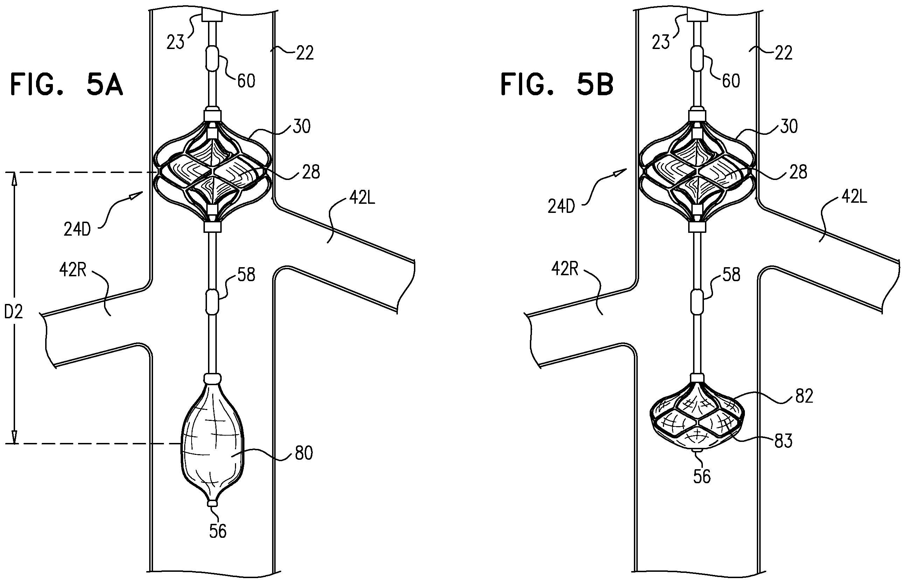

Reference is now made to Figs. 5A-B, which are schematic illustrations of blood-pump catheter 20, the catheter including downstream pump 24D and an occlusion element, such as a balloon 80 (Fig. 5A), or a covered frame 82 (Fig. 5B), in accordance with some applications of the present invention. For some applications, downstream pump is placed inside vena cava 22, downstream of the junctions of the vena cava with all of the subject's renal veins. The downstream pump pumps blood through the vena cava, in the downstream direction, away from the junctions of the vena cava with the renal veins, in the manner described hereinabove. As an alternative to, or in addition to using an upstream pump as described hereinabove, the occlusion element is placed inside the vena cava upstream of the junctions of the vena cava with the subject's renal veins. Typically, the occlusion element is configured to partially occlude the subject's vena cava upstream of the junctions of the vena cava with the subject's renal veins. The occlusion element is configured to partially occlude the subject's vena cava such that, in response to the pumping of the downstream blood pump, there is not a substantial increase of blood flow from the subject's lower body toward the subject heart, but such that a region of low pressure within the vena cava is generated, between the occlusion element and the downstream blood pump, within which the blood pressure is lower than the subject's central venous pressure. Typically, by generating a region of low pressure, blood flow from the renal veins into the vena cava increases, thereby lowering renal blood pressure and enhancing renal perfusion. It is noted that the occlusion element is configured to partially occlude, but not to totally occlude, the vena cava, in such a manner as to generate a region of low pressure within the vena cava, but to allow a substantial flow of blood through the vena cava

When blood-pump catheter 20 is used to enhance blood flow from the renal veins into the subject's vena cava, as described herein, a longitudinal distance D2 between the longitudinal center of the impeller of the downstream pump and the longitudinal center of the occlusion element, measured along the longitudinal axis of the catheter, is typically more than 3 cm (e.g., more than 6 cm), and/or less than 18 cm (e.g., less than 14 cm), e.g., 3-18 cm, or 6-14 cm.

As used in the present application, including in the claims, a "longitudinal axis" of a structure is the set of all centroids of cross-sectional sections of the structure along the structure. Thus the cross-sectional sections are locally perpendicular to the longitudinal axis, which runs along the structure. (If the structure is circular in cross-section, the centroids correspond with the centers of the circular cross-sectional sections.) As used in the present application, including in the claims, the term "longitudinal center" denotes the center of a structure along the direction of the structure's longitudinal axis.

For some applications, the occlusion element is balloon 80, as shown in Fig. 5A. Alternatively or additionally, the occlusion element is covered frame 82, as shown in Fig. 5B. For example, the frame may be a rigid frame made of a shape-memory element (such as nitinol) that is covered with a blood-impermeable material 83 (e.g., polyester, polyurethane, and/or a different polymer).

As described hereinabove, typically, the occlusion element is configured to partially occlude the vena cava upstream of the junctions of the vena cava with the subject's renal veins. For some applications, the diameter to which the occlusion element is expanded is controllable. For example, inflation of the balloon may be controllable, or the stent may be expandable (e.g., by heating the stent, or by applying an electrical current to the stent). For some applications, the extent to which the occlusion element occludes the vena cava is controlled by a control unit (e.g., control unit 52) responsively to the blood pressure detected by blood pressure sensor 56, 58, and/or 60, in response to an input from a different sensor (such as a flow sensor and/or an oxygen-saturation sensor, and/or a thermal flow sensor, e.g., as described with reference to Figs. 22Ai-Cii of WO 14/141284 to 2. Schwammenthal), and/or in response to an input from a user. For some applications, the rate at which pump 24D pumps blood away from the renal veins (e.g., the rate at which impeller 28 of the pump is rotated), as well as the extent to which the occlusion element occludes the vena cava is controlled by a control unit responsively to the blood pressure detected by blood pressure sensor 56, 58, and/or 60, in response to an input from a different sensor (such as a flow sensor and/or an oxygen-saturation sensor, and/or a thermal flow sensor, e.g., as described with reference to Figs. 22Ai-Cii of WO 14/141284 to Schwammenthal), and/or in response to an input from a user.

Although Figs. 5A and 5B show the downstream blood pump and the occlusion element disposed on a catheter that is inserted into the vena cava from above the junctions of the vena cava with the subject's renal veins (e.g., via the subject's subclavian vein or jugular vein), for some applications, the downstream blood pump and the occlusion element are disposed on a catheter that is inserted into the vena cava from below the junctions of the vena cava with the subject's renal veins (e.g., via the subject's femoral vein), *mutatis mutandis.* Alternatively or additionally, the occlusion element is disposed on a first catheter which is inserted into the vena cava from below the junctions of the vena cava with the subject's renal veins (e.g., via the subject's femoral vein), and the downstream blood pump is disposed on a second catheter, which inserted into the vena cava from above the junctions of the vena cava with the subject's renal veins (e.g., via the subject's subclavian vein, or jugular vein).

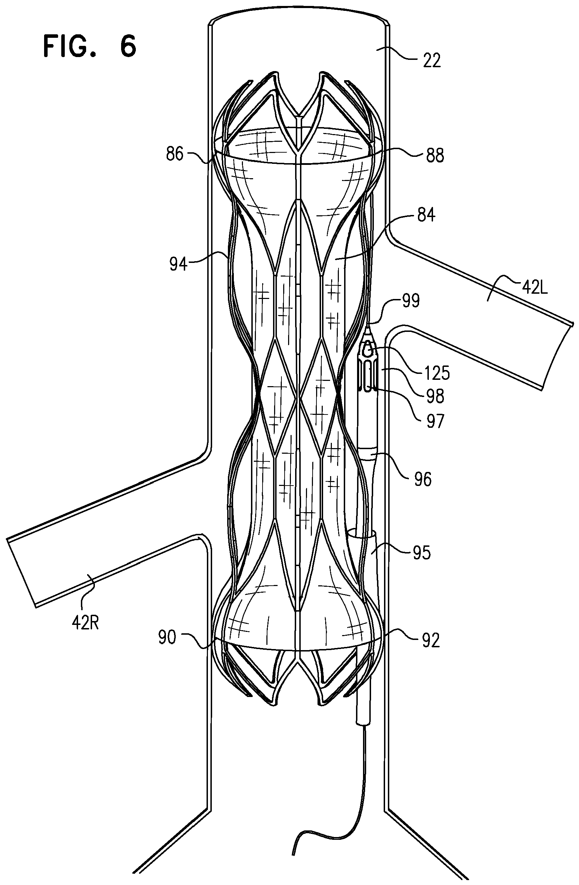

Reference is now made to Fig. 6, which is a schematic illustration of a blood-impermeable sleeve 84 configured to occlude blood flow from a subject's vena cava to the subject's renal veins, as described in WO 14/141284. Typically, the sleeve is placed within the vena cava such that a downstream end 86 of the sleeve is coupled to the wall of the vena cava at a first location 88 that is downstream of all renal veins 42 of the subject (e.g., left and right renal vein in a typical subject that has two renal veins), and such that an upstream end 90 of the sleeve is coupled to a wall of the vena cava at a second location 92 that is upstream of all renal veins of the subject. Thus, the sleeve isolates the blood in the renal veins into a compartment that is separated from blood flow through the center of the vena cava. Typically, a rigid structure, e.g., a stent 94 as shown, is configured to couple the upstream and downstream ends of the sleeve to the vena cava.

A pump 96 is configured to pump blood through inlet holes 97, from a location that is exterior to sleeve 98 (i.e., from the isolated compartment) to a location that is in fluid communication with the interior of the sleeve (e.g., a location within the vena cava upstream or downstream of the sleeve). Thus, the pump pumps blood out of the subject's renal veins and into the subject's vena cava. The sleeve prevents backflow of blood from the vena cava into the renal veins.

For some applications, sleeve 84 and stent 94 are inserted into the subject's vena cava, while a guidewire 99 is disposed inside a pump-accommodating sleeve 95. Subsequent to anchoring sleeve 84 and stent 94 to the vena cava, pump 96 is inserted through the pump-accommodating sleeve, by advancing the pump over the guidewire.

Sleeve 84 and pump 96 are generally as described with reference to Figs. 10A-D of WO 14/141284 to Schwammenthal.

It is noted that the effect of inserting sleeve 84 into the vena cava and activating pump 96 in the described manner is that a low-pressure region is generated within the subject's vena cava, adjacent to junctions of the vena cava with the subject's renal veins, blood pressure within the low-pressure region being lower than central venous pressure of the subject. Similarly, by using blood-pump catheter 20 as described with reference to Figs. 1A-5B, a low-pressure region is generated within the subject's vena cava, adjacent to junctions of the vena cava with the subject's renal veins, blood pressure within the low-pressure region being lower than central venous pressure of the subject. The effect of generating the low-pressure region within the vena cava is typically that blood flow from the renal veins to the vena cava is increased, thereby reducing renal venous pressure, and increasing renal perfusion.

In general, in the specification and in the claims of the present application, the term "proximal" and related terms, when used with reference to a device or a portion thereof, should be interpreted to mean an end of the device or the portion thereof that, when inserted into a subject's body, is typically closer to a location through which the device is inserted into the subject's body. The term "distal" and related terms, when used with reference to a device or a portion thereof, should be interpreted to mean an end of the device or the portion thereof that, when inserted into a subject's body, is typically further from the location through which the device is inserted into the subject's body.

In general, in the specification and in the claims of the present application, the term "downstream" and related terms, when used with reference to a blood vessel, or with reference to a portion of a device that is configured to be placed inside a blood vessel, should be interpreted to mean a location within the blood vessel, or a portion of the device that is intended for placement at a location within the blood vessel, that is downstream, with respect to the direction of antegrade blood flow through the blood vessel, relative to a different location within the blood vessel. The term "upstream" and related terms, when used with reference to a blood vessel, or with reference to a portion of a device that is configured to be placed inside a blood vessel, should be interpreted to mean a location within the blood vessel, or a portion of the device that is intended for placement at a location within the blood vessel, that is upstream with respect to the direction of antegrade blood flow through the blood vessel, relative to a different location within the blood vessel.

It is noted that blood pumps 24U and 24D, the catheters upon which the blood pumps are disposed (e.g., blood-pump catheter 20, catheter 66, and catheter 68), and the occlusion elements described with reference to Figs. 5A-B, and other devices described herein, are generally described as being placed within the subject's vena cava, such that the upstream pump or the occlusion element is disposed upstream of junctions of the vena cava with the subject's renal veins, and the downstream pump is disposed downstream of the junctions of the vena cava with the subject's renal veins. However, it is noted that the scope of the present invention includes placing upstream pump 24U or the occlusion element in any main vein upstream of a tributary venous system, and placing downstream pump 24D downstream of said tributary venous system, and configuring the pump(s) (e.g., via the direction of rotation of impellers of the pumps, or the orientation of the pumps) to generate preferential flow from the tributaries into the main vein, *mutatis mutandis.* For example, the pump(s) could be used to generate flow from the subject's hepatic veins into the subject's vena cava, in order to increase perfusion of the subject's liver, *mutatis mutandis.* For some applications, the upstream pump or the occlusion element is placed within a main vein upstream of two or more tributary venous systems into the main vein (e.g., within the vena cava upstream of the renal venous system and the hepatic venous system). The downstream pump is placed downstream of the two or more tributary venous systems. The pump(s) are configured to generate preferential flow from both of the tributary venous systems into the main vein by pumping blood through the main vein, in the manner described herein.

For such applications, upstream pump 24U or the occlusion element is placed in a main vein upstream of a tributary venous system, and downstream pump 24D is placed downstream of said tributary venous system, and the pump(s) are configured (e.g., via the direction of rotation of impellers of the pumps, or the orientation of the pumps) to reduce flow from the tributaries into the main vein. For some such applications, the blades of the downstream impeller are oriented such that, as the downstream impeller is rotated, the downstream impeller pumps in the upstream direction (toward the junction between the tributary system and the main vein). The blades of the upstream impeller are oriented such that, as the upstream impeller rotates is rotated, the upstream impeller pumps in the downstream direction (toward the junction between the tributary system and the main vein).

For some applications, the upstream and downstream pumps 24U and 24D, the catheter(s) upon which the blood pumps are disposed (e.g., blood-pump catheter 20, catheter 66, and catheter 68), and/or the occlusion elements described with reference to Figs. 5A-B, and other devices described herein, are placed within a main artery upstream and downstream of bifurcations of the artery with one or more branching arterial systems that branch from the main artery and supply a given organ, *mutatis mutandis.* For such applications, the upstream pump is typically configured to pump in the downstream direction (toward the bifurcations) and the downstream pump is configured to pump in the upstream direction (toward the bifurcations), such that blood flow into the branching arterial system is increased, thereby increasing perfusion of the organ. Alternatively or additionally, the occlusion element is placed downstream of the bifurcations of the artery with the one or more arterial systems and is configured to partially occlude the artery downstream of the bifurcations. For example, the upstream pump may be placed in the subject's aorta upstream of the subject's renal arteries and the downstream pump may be placed in the subject's aorta downstream of the subject's renal arteries, the pumps acting to pump blood into the renal arteries and toward the subject's kidneys. For some applications, upstream and downstream pumps, and/or occlusion elements are placed on both the arterial and venous sides of the subject's body in order to increase perfusion of a given organ or set of organs, in the manner described herein.

Although some applications of the present invention are described with reference to blood pumps 24D and 24U, according to which the blood pumps include impellers, the scope of the present invention includes using any other type of pump for pumping blood in the manner described herein, *mutatis mutandis.* For example, a roller pump, an Archimedes screw pump, a centrifugal pump, a pneumatic pump, and/or a compression pump may be used.

The scope of the present invention, as defined in the appended claims, includes combining any of the apparatus and methods described herein with any of the apparatus and methods described in one or more of the following 2. applications:
International Patent Application PCT/IL2014/050289 to Schwammenthal (published as WO 14/141284), filed March 13, 2014, entitled "Renal pump," which claims priority from (a) US Provisional Patent Application 61/779,803 to Schwammenthal, filed March 13, 2013, entitled "Renal pump," and (b) US Provisional Patent Application 61/914,475 to Schwammenthal, filed December 11, 2013, entitled "Renal pump;" and
International Patent Application PCT/IL2013/050495 to Tuval (published as WO 13/183060), filed June 06, 2013, entitled "Prosthetic renal valve," which claims priority from US Provisional Patent Application 61/656,244 to Tuval, filed June 06, 2012, entitled "Prosthetic renal valve."

## Claims

1. Apparatus comprising:
a catheter (20) configured to be placed inside a blood vessel of a subject;
a blood pump (24D) disposed on the catheter (20); and
an occlusion element (80, 82) disposed on the catheter (20), and configured to partially occlude the subject's blood vessel,
longitudinal centers of the blood pump (24D) and the occlusion element (80, 82) being separated from one another by a distance of at least 3 cm, the distance being measured along a longitudinal axis of the catheter (20),
**characterized in that**:
the blood pump (24D) and the occlusion element (80, 82) are configured to generate a region within the blood vessel that is of lower blood pressure than elsewhere within the blood vessel by the blood pump (24D) pumping away from a region of the blood vessel between the blood pump (24D) and the occlusion element (80, 82) in a downstream direction.

2. The apparatus according to claim 1, wherein the blood pump (24D) comprises an impeller (28) configured to pump blood through the subject's blood vessel by rotating.

3. The apparatus according to claim 2, further comprising a cage (30), the impeller (28) being disposed inside the cage (30), and the cage (30) being configured to maintain a separation between the impeller (28) and an inner wall of the blood vessel.

4. The apparatus according to claim 1, wherein the catheter (20) is configured to be placed within a vena cava of a subject such that the blood pump (24D) is disposed downstream of junctions of the vena cava with all renal veins of the subject, and such that the occlusion element (80, 82) is disposed upstream of junctions of the vena cava with all renal veins of the subject.

5. The apparatus according to claim 4, wherein the blood pump (24D) is configured to lower pressure within the subject's renal veins by pumping blood through the vena cava in a downstream direction.

6. The apparatus according to claim 4, wherein the catheter (20) is configured to be placed within the subject's vena cava by being inserted via a vein of the subject selected from the group consisting of: a subclavian vein, a jugular vein, and a femoral vein.

7. The apparatus according to claim 4, wherein the blood pump (24D) comprises an impeller (28) configured to pump blood through the vena cava by rotating.

8. The apparatus according to claim 7, further comprising a cage (30), the impeller (28) being disposed inside the cage (30), and the cage (30) being configured to maintain a separation between the impeller (28) and an inner wall of the vena cava.

9. The apparatus according to claim 1, wherein the occlusion element (80, 82) is configured to partially occlude the subject's vena cava such that, in response to the pumping of the blood pump (24D) in the downstream direction, there is not a substantial increase of blood flow from a lower body of the subject toward a heart of the subject, but such that a region of low pressure within the vena cava is generated, between the occlusion element and the downstream blood pump, within which blood pressure is lower than central venous pressure of the subject.

10. The apparatus according to claim 1, wherein the catheter (20) is configured to be placed within a main vein of a subject into which blood flows from a tributary venous system such that:
the blood pump (24D) is placed in the main vein, downstream of the tributary venous system; and
the occlusion element (80, 82) is placed in the main vein, upstream of the tributary venous system.

11. The apparatus according to claim 1, wherein the occlusion element (80, 82) comprises a balloon.

12. The apparatus according to claim 1, wherein the occlusion element (80, 82) comprises a frame covered with a blood impermeable material.

13. The apparatus according to claim 1, further comprising a control unit configured to control a diameter to which the occlusion element (80, 82) is expanded.

14. The apparatus according to claim 1, wherein the occlusion element (80, 82) is configured to partially occlude, but not to totally occlude, the blood vessel, in such a manner as to generate a region of low pressure within the region of the blood vessel between the blood pump (24D) and the occlusion element (80, 82), but to allow a substantial flow of blood through the blood vessel.

## Patentansprüche

1. Vorrichtung, die Folgendes umfasst:
einen Katheter (20), der dafür konfiguriert ist, innerhalb eines Blutgefäßes eines Subjekts platziert zu werden,
eine Blutpumpe (24D), die an dem Katheter (20) angeordnet ist, und
ein Verschlusselement (80, 82), das an dem Katheter (20) angeordnet und dafür konfiguriert ist, das Blutgefäß des Subjekts teilweise zu verschließen,
wobei Längsmitten der Blutpumpe (24D) und des Verschlusselements (80, 82) durch einen Abstand von mindestens 3 cm voneinander getrennt sind, wobei der Abstand entlang einer Längsachse des Katheters (20) gemessen wird,
**dadurch gekennzeichnet, dass**:
die Blutpumpe (24D) und das Verschlusselement (80, 82) dafür konfiguriert sind, dadurch, dass die Blutpumpe (24D) von einem Bereich des Blutgefäßes zwischen der Blutpumpe (24D) und dem Verschlusselement (80, 82) weg in einer Richtung stromabwärts pumpt, einen Bereich innerhalb des Blutgefäßes zu erzeugen, der einen niedrigeren Blutdruck als an anderer Stelle innerhalb des Blutgefäßes aufweist.

2. Vorrichtung nach Anspruch 1, wobei die Blutpumpe (24D) ein Laufrad (28) umfasst, das dafür konfiguriert ist, durch Drehen Blut durch das Blutgefäß des Subjekts zu pumpen.

3. Vorrichtung nach Anspruch 2, die ferner einen Käfig (30) umfasst, wobei das Laufrad (28) innerhalb des Käfigs (30) angeordnet ist und der Käfig (30) dafür konfiguriert ist, eine Trennung zwischen dem Laufrad (28) und einer inneren Wand des Blutgefäßes aufrechtzuerhalten.

4. Vorrichtung nach Anspruch 1, wobei der Katheter (20) dafür konfiguriert ist, derart innerhalb einer Hohlvene eines Subjekts angeordnet zu werden, dass die Blutpumpe (24D) stromabwärts von Verbindungen der Hohlvene mit allen Nierenvenen des Subjekts angeordnet ist, und derart, dass das Verschlusselement (80, 82) stromaufwärts von Verbindungen der Hohlvene mit allen Nierenvenen des Subjekts angeordnet ist.

5. Vorrichtung nach Anspruch 4, wobei die Blutpumpe (24D) dafür konfiguriert ist, durch Pumpen von Blut durch die Hohlvene in einer Richtung stromabwärts den Druck innerhalb der Nierenvenen des Subjekts abzusenken.

6. Vorrichtung nach Anspruch 4, wobei der Katheter (20) dafür konfiguriert ist, dadurch innerhalb der Hohlvene des Subjekts platziert zu werden, dass er über eine Vene des Subjekts eingesetzt wird, die aus der Gruppe ausgewählt ist, die aus einer Schlüsselbeinvene, einer Drosselvene und einer Oberschenkelvene besteht.

7. Vorrichtung nach Anspruch 4, wobei die Blutpumpe (24D) ein Laufrad (28) umfasst, das dafür konfiguriert ist, durch Drehen Blut durch die Hohlvene zu pumpen.

8. Vorrichtung nach Anspruch 7, die ferner einen Käfig (30) umfasst, wobei das Laufrad (28) innerhalb des Käfigs (30) angeordnet ist und der Käfig (30) dafür konfiguriert ist, eine Trennung zwischen dem Laufrad (28) und einer inneren Wand der Hohlvene aufrechtzuerhalten.

9. Vorrichtung nach Anspruch 1, wobei das Verschlusselement (80, 82) dafür konfiguriert ist, die Hohlvene des Subjekts derart teilweise zu verschließen, dass es, in Reaktion auf das Pumpen der Blutpumpe (24D) in der Richtung stromabwärts, keine wesentliche Zunahme eines Blutflusses von einem Unterkörper des Subjekts hin zu einem Herzen des Subjekts gibt, aber derart, dass innerhalb der Hohlvene, zwischen dem Verschlusselement und der Stromabwärts-Blutpumpe, ein Bereich mit niedrigem Druck erzeugt wird, innerhalb dessen der Blutdruck niedriger ist als der zentrale Venendruck des Subjekts.

10. Vorrichtung nach Anspruch 1, wobei der Katheter (20) dafür konfiguriert ist, derart innerhalb einer Hauptvene eines Subjekts, in die Blut aus einem sekundär Venensystem fließt, angeordnet zu werden, dass:
die Blutpumpe (24D) in der Hauptvene, stromabwärts von dem zufließenden Venensystem, platziert ist, und
das Verschlusselement (80, 82) in der Hauptvene, stromaufwärts von dem zufließenden Venensystem, platziert ist.

11. Vorrichtung nach Anspruch 1, wobei das Verschlusselement (80, 82) einen Ballon umfasst.

12. Vorrichtung nach Anspruch 1, wobei das Verschlusselement (80, 82) einen Rahmen umfasst, der mit einem blutundurchlässigen Material bedeckt ist.

13. Vorrichtung nach Anspruch 1, die ferner eine Steuereinheit umfasst, die dafür konfiguriert ist, einen Durchmesser zu steuern, bis zu dem das Verschlusselement (80, 82) ausgedehnt wird.

14. Vorrichtung nach Anspruch 1, wobei das Verschlusselement (80, 82) dafür konfiguriert ist, das Blutgefäß auf eine solche Weise teilweise zu verschließen, aber nicht vollständig zu verschließen, dass ein Bereich mit niedrigem Druck innerhalb des Bereichs des Blutgefäßes zwischen der Blutpumpe (24D) und dem Verschlusselement (80, 82) erzeugt wird, aber ein wesentlicher Blutfluss durch das Blutgefäß zugelassen wird.

## Revendications

1. Appareil, comprenant :
un cathéter (20) configuré pour être placé à l'intérieur d'un vaisseau sanguin d'un sujet ;
une pompe à sang (24D) disposée sur le cathéter (20) ; et
un élément d'occlusion (80, 82) disposé sur le cathéter (20) et configuré pour occlure partiellement le vaisseau sanguin du sujet ;
des centres longitudinaux de la pompe à sang (24D) et de l'élément d'occlusion (80, 82) étant séparés l'un de l'autre d'une distance d'au moins 3 cm, la distance étant mesurée le long d'un axe longitudinal du cathéter (20) ;
**caractérisé en ce que** :
la pompe à sang (24D) et l'élément d'occlusion (80, 82) sont configurés pour générer une région à l'intérieur du vaisseau sanguin ayant une pression artérielle inférieure à celle existant dans d'autres endroits du vaisseau sanguin par suite du pompage par la pompe à sang (24D) à l'écart d'une région du vaisseau sanguin entre la pompe à sang (24D) et l'élément d'occlusion (80, 82) dans une direction allant vers l'aval.

2. Appareil selon la revendication 1, dans lequel la pompe à sang (24D) comprend une roue (28) configurée pour pomper du sang à travers le vaisseau sanguin du sujet par rotation.

3. Appareil selon la revendication 2, comprenant en outre une cage (30), la roue (28) étant disposée à l'intérieur de la cage (30), et la cage (30) étant configurée pour maintenir une séparation entre la roue (28) et une paroi interne du vaisseau sanguin.

4. Appareil selon la revendication 1, dans lequel le cathéter (20) est configuré pour être placé à l'intérieur d'une veine cave d'un sujet, de sorte que la pompe à sang (24D) est disposée en aval des jonctions de la veine cave avec toutes les veines rénales du sujet, et de sorte que l'élément d'occlusion (80, 82) est disposé en amont des jonctions de la veine cave avec toutes les veines rénales du sujet.

5. Appareil selon la revendication 4, dans lequel la pompe à sang (2D) est configurée pour réduire la pression dans les veines rénales du sujet en pompant du sang à travers la veine cave dans une direction allant vers l'aval.

6. Appareil selon la revendication 4, dans lequel le cathéter (20) est configuré pour être placé à l'intérieur de la veine cave du sujet par insertion à travers une veine du sujet sélectionnée dans le groupe constitué d'une veine sous-clavière, d'une veine jugulaire et d'une veine fémorale.

7. Appareil selon la revendication 4, dans lequel la pompe à sang (24D) comprend une roue (28) configurée pour pomper du sang à travers la veine cave par rotation.

8. Appareil selon la revendication 7, comprenant en outre une cage (30), la roue (28) étant disposée à l'intérieur de la cage (30), et la cage (30) étant configurée pour maintenir une séparation entre la roue (28) et une paroi interne de la veine cave.

9. Appareil selon la revendication 1, dans lequel l'élément d'occlusion (80, 82) est configuré pour occlure partiellement la veine cave du sujet, de sorte que, en réponse au pompage par la pompe à sang (24D) dans la direction allant vers l'aval, il n'y a pas d'accroissement substantiel de l'écoulement du sang depuis la partie inférieure du corps du sujet vers un cœur du sujet, mais de sorte à générer une région à basse pression dans la veine cave, entre l'élément d'occlusion et la pompe à sang en aval, dans laquelle la pression artérielle est inférieur à la pression veineuse centrale du sujet.

10. Appareil selon la revendication 1, dans lequel le cathéter (20) est configuré pour être placé dans une veine principale d'un sujet dans laquelle du sang s'écoule à partir d'un système veineux secondaire, de sorte que :
la pompe à sang (24D) est placée dans la veine principale, en aval du système veineux secondaire ; et
l'élément d'occlusion (80, 82) est placé dans la veine principale, en amont du système veineux secondaire.

11. Appareil selon la revendication 1, dans lequel l'élément d'occlusion (80, 82) comprend un ballonnet.

12. Appareil selon la revendication 1, dans lequel l'élément d'occlusion (80, 82) comprend un cadre recouvert d'un matériau imperméable au sang.

13. Appareil selon la revendication 1, comprenant en outre une unité de commande configurée pour contrôler un diamètre auquel l'élément d'occlusion (80, 82) est étendu.

14. Appareil selon la revendication 1, dans lequel l'élément d'occlusion (80, 82) est configuré pour occlure partiellement, mais pas pour occlure complètement le vaisseau sanguin, de manière à générer une région à base pression dans la région du vaisseau sanguin entre la pompe à sang (24D) et l'élément d'occlusion (80, 82), mais pour permettre un écoulement sanguin substantiel à travers le vaisseau sanguin.
